# EUROPEAN PATENT APPLICATION

(11) **EP 3 252 167 A1**
(43) Date of publication of application: **06.12.2017**
(21) Application number: 16743509.8
(22) Date of filing: 29.01.2016
(51) Int. Cl.: C12Q 1/68, C12M 1/00, C12N 15/09

(54) **VISUALLY DETERMINABLE GENETIC TESTING**

(30) Priority: 30.01.2015 JP 2015017396
(71) Applicant: Kurashiki Boseki Kabushiki Kaisha, Kurashiki-shi, Okayama 710-0054 (JP)
(72) Inventor: YAMASHITA, Hiroki, Neyagawa-shi, Osaka 572-0823 (JP); KOGOH, Kazuhiko, Neyagawa-shi, Osaka 572-0823 (JP); OMURA, Shuichi, Neyagawa-shi, Osaka 572-0823 (JP)
(74) Representative: Hoffmann Eitle
(86) International application number: PCT/JP2016/052600
(87) International publication number: WO 2016/121906

(57) **Abstract**

The purpose of the present invention is to provide a DNA detection technique by which testing can be simply and accurately performed at clinical sites and the like. The present invention provides a gene analysis method which employs, as a detection sample, a single strand nucleotide product that is ligated and amplified, and which can be performed with the naked eye in a more simple and rapid way.

## Description

### Technical Field

The present invention relates to a technique for amplifying a single-stranded DNA of target gene for a visually determinable genetic testing.

### Background Art

Genetic tests that analyze and determine the base sequence of a specimen have been widely used in the fields of basic research, medical, food, and the like. More specifically, genetic tests have been used for detection of mutation, determination of genetic polymorphism (for humans and animals, refers to one having a change frequency of 1% or more), detection of pathogenic bacteria, inspection of the presence or absence of genetic modification, and the like. For example, genetic polymorphism determination has begun to be used at medical sites and the like since side effects and effects of drugs can be predicted in advance.

Genetic tests are typically performed by using the TaqMan or QP method using a real-time PCR instrument, a method using a DNA microarray (DigiTag method, etc.), or the like. However, all of these methods require skill acquisition and expensive fluorescence detection device. Thus, while these methods are used in an entrusted analysis, it is currently difficult to be used at medical sites and the like.

Further, methods for detecting a target nucleic acid on a solid phase on which an oligonucleotide is immobilized are disclosed (Patent Document 1, Patent Document 2). The term "solid phase" as used herein refers to a so-called "strip" to which an oligonucleotide is bound in a linear or spot shape on a membrane such as a nitrocellulose membrane or a nylon membrane on which an oligonucleotide can be immobilized, and a specific example is one called PAS (Printed Array Strip). According to the principle of nucleic acid chromatography, it is possible to develop a DNA product prepared by PCR on this strip and to detect it by hybridization. In this method, the prepared DNA product is of double-stranded and is characterized by adding a single tag to the end of one strand of the DNA product. However, since the target double-stranded DNA sequence is amplified by PCR, the method is high in sensitivity (reactivity), but has a problem that nonspecific amplification products such as primer dimers tend to be generated, and the accuracy (precision) is low.

### Prior Art Documents

### Patent Documents

Patent Document 1: JP 5503021 B
Patent Document 2: WO 2012/070618 A
Patent Document 3: JP 3103806 B
Patent Document 4: JP 2006-101844 A
Patent Document 5: JP H06-36760 B
Patent Document 6: JP 3330599 B

### Summary of Invention

### Problems to be Solved by Invention

An object of the present invention is to provide a technique of a genetic analysis that enables to simply perform a test with accuracy even at medical sites and the like.

In the conventional technique by a strip, a target DNA is amplified, and the amplified DNA product is "developed" on the strip, then the oligonucleotide on the strip and the DNA product undergo hybridization, so that determination can be made. In this technique, the target DNA is amplified in a large amount by PCR, and the DNA product is developed on the strip. Therefore, amplification by PCR has been essential for detecting the DNA product on the strip. However, in the DNA product amplified by PCR, there have been problems that nonspecific DNA products such as primer dimers are often generated, and DNA that is not originally detected is detected on the strip, resulting in erroneous determination.

When SNP is detected with a strip, a method has been used which amplifies double-stranded DNA corresponding to SNP by matching the 3' end of the primer used for PCR with the base corresponding to the SNP. However, hybridization of the primer has sometimes occurred erroneously in the PCR amplification step of the SNP, resulting in a problem that the accuracy is further lowered.

Furthermore, in the detection using a strip, a detection method using a single-stranded DNA sequence is conceivable. For example, it is considered to obtain a single-stranded DNA product by separating a double-stranded DNA product amplified by PCR, in which a single tag is added to one end of the single-stranded DNA and a labeling substance is added to the other end, into single-stranded DNAs by thermal denaturation. However, in this method, the above-mentioned problems occur and the accuracy is lowered in PCR. Further, the DNA product is separated by thermal denaturation, so that when the DNA product is developed on the strip, a part of the separated single-stranded DNA has returned to double-stranded DNA, resulting in a problem that the sensitivity is further lowered.

For this reason, the establishment of DNA detection technique that can simply perform a test with high accuracy at medical sites and the like is one of important subjects. In particular, it is focused on a genetic testing techniques which use strips and which can simply perform a test with high accuracy at medical sites and the like without requiring a large-scale apparatus or the like.

In order to solve the above problems, the present inventors have focused on not amplifying a nonspecific double-stranded DNA product such as a primer dimer by not preparing a double-stranded DNA product in a sample used for tests. Therefore, development of a technique of gene detection with high accuracy has been studied by using a single-stranded DNA product amplified by ligation as a detection sample.

A technique for obtaining a single-stranded DNA has conventionally existed. As well-known techniques, there are techniques such as Digitag method (Patent Document 3 and Patent Document 4) and LCR (Patent Document 5 and Patent Document 6). However, it has been found that the oligonucleotide used for the reaction nonspecifically binds to a nucleic acid sample in these techniques, and it is erroneously determined as a false positive on the strip.

A process of the Digitag method, which is a technique for obtaining a single-stranded DNA, will be described. A target gene sequence of a nucleic acid sample is amplified by PCR. The obtained PCR products are separated by heat denaturation. Then, a ligation reaction is performed by using a query oligo and a common oligo to obtain a ligation product of single-stranded DNA. By using the obtained single-stranded DNA as a template, it is amplified again by PCR. The obtained PCR product is made into a single strand by heat denaturation, and the single strand was dispensed on a microarray. The oligonucleotide probe on the microarray hybridizes to the single-stranded DNA, and the fluorescence emitted from the hybridized single-stranded DNA is read and detected by the device. At this time, the obtained single-stranded DNA is composed of primers with two kinds of fluorescent labels, green and red. With these fluorescent lights, green and red, and yellow in which green and red are mixed are made. For this reason, in the Digitag method, even when the DNA of the other fluorescent label is hybridized in the detection of monochromatic green or red fluorescence, the DNA is not detected because the amount of the DNA is very small. Therefore, it is not a problem even when a slight amount of a dimer is generated.

However, when the single-stranded DNA obtained by the Digitag method is developed on a strip, even if a gene amount of a dimer is small, the dimer appears on the reaction as a line, and it may be detected as a false positive. Therefore, although the Digitag method is excellent as a technique for obtaining a single-stranded DNA, it cannot be used with a strip from the viewpoint of accuracy.

Next, a process of LCR reaction, which is a technique for obtaining a single-stranded DNA, will be described. A target gene sequence of a nucleic acid sample is amplified by PCR. After the PCR, a query oligo and a common oligo, a C query oligo and a C common oligo complementary to the query oligo and the common oligo, ligase and the like are mixed. After the obtained PCR products are separated by heat denaturation, a ligation reaction is performed by using the query oligo and the common oligo to obtain a ligation product of single-stranded DNA. Furthermore, ligation products that are complementary to the obtained ligation products are obtained by the C query oligo and the C common oligo. By using the obtained ligation product, an F query oligo and an F common oligo, a ligation product is obtained. This process is repeated, so that a large amount of single-stranded DNA can be obtained.

However, when the single-stranded DNA obtained by the LCR is developed on a strip, it may be detected as a false positive. This is because a large amount of single-stranded DNA is generated, thus mismatch hybridization occurs between the probe and the template, and a ligation product is generated as an unintended single-stranded DNA that can be a false positive. Furthermore, mismatch hybridization occurs between the probes, so that further unintended single-stranded DNA is generated. Therefore, although the method is excellent as a technique for obtaining a single-stranded DNA, it cannot be used with a strip from the viewpoint of accuracy.

Furthermore, when the kinds of genes to be amplified by multiplex PCR are increased as in the Digitag method, easily reactable genes are preferentially amplified, the amplification efficiency of hardly reactable genes may be deteriorated. Therefore, the nucleic acid sample to be originally detected is not detected, and it may be erroneously determined as a false negative.

### Means for Solving the Problems

In order to solve these problems, the present inventors have focused on gene amplification treatment without using complementary strand DNA, unlike PCR or LCR, and found a method capable of simply acquiring a single-stranded DNA with high accuracy, and thus the present invention has been completed. Conventionally, it has been pointed out that the production of single-stranded DNA has a problem that the amplification efficiency of DNA is worse than that in PCR, and sensitivity is low. However, contrary to this point, this technique realizes a sensitivity sufficient for a simple test in clinical sites.

Specifically, the present invention provides the followings:
(1) a genetic analysis method comprising hybridizing the following (i) and (ii) and making the hybridized product visually detectable:
   (i) an oligonucleotide probe immobilized on a strip;
   (ii) a single-stranded ligation product amplified by subjecting the following (a) and (b) to ligation treatment through one or a plurality of thermal cycling treatments using a nucleic acid sample as a template:
      (a) a query oligo comprising a first single-stranded oligonucleotide complementary to said nucleic acid sample and a tag sequence capable of hybridizing with said probe or a labeling moiety;
      (b) a common oligo comprising a second single-stranded oligonucleotide complementary to said nucleic acid sample and adjacent to said first single-stranded oligonucleotide and a tag sequence or a labeling moiety;
(2) the method according to (1), further comprising performing a cycling ligation reaction;
(3) the method according to (1) or (2), wherein the nucleic acid sample and the oligonucleotide are DNA;
(4) the method according to any one of (1) to (3), wherein the strip is a chromatography main body having a probe region in which a plurality of probes is immobilized at different positions and a position marker region at a position different from the probe region;
(5) the method according to any one of (1) to (4), for detecting a gene difference;
(6) the method according to any one of (1) to (5), for detecting a gene polymorphism; and
(7) a kit for a genetic analysis method according to any one of (1) to (6), comprising
   a strip on which oligonucleotide probe is immobilized,
   a single-stranded oligonucleotide comprising a sequence complementary to a nucleic acid sample and a tag sequence capable of hybridizing with said probe, and
   a single-stranded oligonucleotide comprising a sequence complementary to said nucleic acid sample and adjacent to said first single-stranded oligonucleotide and a labeling moiety or a tag sequence.

### Effects of Invention

The present invention enables to visually perform a genetic analysis more simply and quickly.

### Brief Description of Drawings

Fig. 1 is a diagram showing a treatment of a sample according to the present invention.
Fig. 2 is a diagram showing a confirmation result of fragments obtained by amplifying a sample of Example 1 by PCR.
Fig. 3 is a schematic diagram of PAS (Printed Array Strip) used in Example 1.
Fig. 4 is a diagram showing detection results of Example 1.
Fig. 5 is a diagram showing specific examples of temperature changes in an ordinary ligation.
Fig. 6 is a diagram showing specific examples of temperature changes in a cycling ligation reaction.
Fig. 7 is a diagram showing a treatment (cycling ligation reaction) of a sample according to the present invention.
Fig. 8 is a diagram showing a confirmation result of fragments obtained by amplifying a sample of Example 2 by PCR.
Fig. 9 is a schematic diagram of PAS used in Examples 2 and 5.
Fig. 10 is a diagram showing detection results of Example 2.
Fig. 11 is a diagram showing a confirmation result of fragments obtained by amplifying a sample of Example 3 by PCR.
Fig. 12 is a schematic diagram of PAS used in Examples 3 and 4.
Fig. 13 is a diagram showing detection results of Example 3.
Fig. 14 is a diagram showing a confirmation result of fragments obtained by amplifying a sample of Example 4 by PCR.
Fig. 15 is a diagram showing detection results of Example 4.
Fig. 16 is a diagram showing detection results of Example 5.

### Mode for Carrying Out the Invention

In one aspect, the present invention relates to a method of visually detectable gene analysis, such as analysis of plurality of genetic polymorphisms, of nucleic acid sample. More specifically, the present invention relates to a method which enables to visually detect a target nucleic acid by hybridizing a ligation product of single-stranded DNA containing a tag sequence and a labeling moiety prepared by using a nucleic acid sample to an oligonucleotide probe bound to a solid-phase carrier.

A genetic testing method of the present invention may be used for an analysis of sequence of any gene. For example, the method may be used for genetic analysis of bacteria, viruses, plants, animals including humans, and animals other than humans, for example, it enables to detect gene mutations such as variations, genetic polymorphisms, and mutations. More specifically, the method of the present invention may be applied to, for example, detection and type determination of HPV, dengue fever, and influenza virus; determination of species of animals and plants; detection of genetically modified crops; and detection of viral drug resistant strain gene, drug resistant strain gene, single nucleotide polymorphism (SNP), microsatellite, substitution, deletion, insertion, and the like. For example, a genetic testing method of the present invention may be used for detecting genetic polymorphisms.

### (Nucleic acid sample)

A nucleic acid sample, which is subjected to the present invention, is a molecule in which nucleotides are polymerized, and examples of the nucleic acid sample include oligonucleotides, polypeptides, and the like. Moreover, the nucleic acid sample includes all kinds of single-stranded or double-stranded DNA and the like. Further, included are those formed solely of naturally-occurring nucleotides and those partially containing non naturally-occurring bases, nucleotides, and nucleosides, and synthetic nucleic acids. Typically, the nucleic acid sample is DNA.

Also, the nucleic acid sample, which is subjected to the present invention, may be obtained by a method well known to those skilled in the art, for example, extraction, or the like with use of a sample derived from various animals and plants, for example, blood, urine, nasal discharge, saliva, tissues, cells, seeds, plant tissues, plant cultures, etc. and a sample derived from microorganisms. The obtained nucleic acid may or may not be purified prior to treatment. The nucleic acid sample may also be obtained by artificial synthesis. Preferably, the nucleic acid sample is amplified by PCR before a ligation treatment described later. In this case, the primers used for PCR may be properly selected, designed and prepared by those skilled in the art depending on the target sample.

It is not always a necessary step to amplify a specific site of the nucleic acid sample by PCR, but it enables to obtain an amplification product with higher accuracy in the ligation treatment described later.

In PCR, multiplex PCR capable of simultaneously amplifying a plurality of genes may be performed. The number of genes that may be amplified is 2 to 15, preferably 3 to 10, and further preferably 4 to 8. When PCR is performed with the number of genes exceeding 16, many dimers are generated, so that the accuracy is lowered, and it is not possible to use the PCR easily at the sites and the like.

The Tm value of PCR is preferably 60 to 80°C, further preferably 65 to 75°C. By setting the Tm value at a relatively high temperature, the nucleic acid sample is specifically annealed with the primer, and mismatch of multiplex PCR is reduced.

### (Solid-phase Carrier)

A solid-phase carrier on which an oligonucleotide probe is immobilized is used in the present invention. Preferably, a "strip" that is an array in which an oligonucleotide probe is bound onto a membrane is used as the solid-phase carrier. Such a solid-phase carrier may be prepared before performing the genetic analysis method. Commercially available product may be used as such a solid-phase carrier. Preferably, the solid-phase carrier has a probe region in which a plurality of probes is immobilized at different positions. In addition, preferably, the solid-phase carrier has a position marker region at a position different from the probe region. The oligonucleotide probe immobilized on the solid-phase carrier is also herein referred to as a detection probe.

A strip used is a chromatography main body capable of moving a developing solvent, and is not particularly limited as long as the strip may be used in the genetic analysis of the present invention. Examples of the chromatography main body include those having silica, a membrane material and the like immobilized on a substrate such as glass or plastic by coating or adhesion, those having a membrane material such as nitrocellulose immobilized on a paper piece, and the like. An example of a preferable commercial product of such a strip is PAS (manufactured by TBA).

A length of the detection probe is not particularly limited and may be properly selected by those skilled in the art depending on the target nucleic acid sample. A preferred example is 20 bases or more and 50 bases or less. The detection probe is preferably DNA.

### (Query oligo and common oligo)

The method for genetic analysis of the present invention includes preparing a query oligo and a common oligo.

The query oligo as used herein refers to one containing a first single-stranded oligonucleotide complementary to the nucleic acid sample and optionally a tag sequence hybridizable to a detection probe, or optionally a labeling moiety. In the present invention, the query oligo contains a tag sequence or a labeling moiety. Typically, a tag sequence or labeling moiety is bound to the 5'-end of the first single-stranded oligonucleotide. A spacer sequence may be bound between the tag sequence and the first single-stranded oligonucleotide, or the tag sequence and the first single-stranded oligonucleotide may be directly bonded without a spacer sequence.

The common oligo as used herein refers to one containing a second single-stranded oligonucleotide that is complementary to the nucleic acid sample and that is adjacent to the first single-stranded oligonucleotide, and optionally a labeling moiety, or optionally a tag sequence hybridizable to a detection probe. In one embodiment of the present invention, the common oligo contains a labeling moiety or a tag sequence. Typically, a labeling moiety or tag sequence is bound to the 3'-end of the second single-stranded oligonucleotide. Also, a phosphoric acid may or may not be added to the 5'-end of the second single-stranded oligonucleotide. However, when a phosphoric acid is not added, a step of adding a phosphoric acid to the 5'-end before the ligation reaction described later may be performed.

In a preferred embodiment of the present invention, the query oligo contains a tag sequence, and the common oligo contains a labeling moiety. In other preferred embodiments, the query oligo contains a labeling moiety, and the common oligo contains a tag sequence. In yet other preferred embodiments, the query oligo contains a tag sequence, and the common oligo contains a tag sequence.

The labeling moiety is not particularly limited as long as it may detect a single-stranded oligonucleotide hybridized to the detection probe. Examples of the labeling moiety include fluorescent substances such as fluorescein, Texas red, rhodamine and green fluorescent protein; luminous substances such as luminol and acridinium derivatives; radioactive substances such as ³H, ¹²⁵I, ³⁵S, ¹⁴C and ³²P; magnetic substances such as magnetic beads; enzymes such as peroxidase, alkali phosphatase, β-glucuronidase, β-D-glucosidase and β-D-galactosidase; colorants such as pigments and dyes; affinity labels such as biotin, avidin and streptavidin; and the like. The method of the present invention may be used for visual inspection, and it is preferable to use a labeling moiety such as a fluorescent substance, a luminescent substance, or a colorant. It is also preferable to perform detection by using biotin as the labeling moiety and binding avidin or streptavidin to which colored beads or the like are bound to the biotin. In addition, detection may be realized by using an antibody and/or a hapten as the labeling moiety and by binding to the labeling moiety one or two or more kinds selected from the group consisting of fluorescence, radioactivity, magnetism, enzyme, phosphorescence, chemiluminescence and coloration as a labeling substance. A hapten refers to a substance that binds to an antibody alone, but itself has no ability to cause an immune response, and examples thereof include biotin, digoxigenin, FITC, and the like.

A length of each of the first single-stranded oligonucleotide and the second single-stranded oligonucleotide may be appropriately set by those skilled in the art depending on the target sample. For example, the first single-stranded oligonucleotide is 10 to 60 bases, and the second-single stranded oligonucleotide is 20 to 70 bases. Preferably, the first single-stranded oligonucleotide is 12 to 40 bases, and the second-single stranded oligonucleotide is 20 to 50 bases.

When the method of genetic analysis of the present invention is used for a detection of gene mutation, the mutated portion may be contained in either the query oligo or the common oligo. Preferably, the mutated portion is contained in the query oligo. More preferably, the mutated portion is located at the 3'-end of the query oligo.

Preferable concentrations of the query oligo and the common oligo may be properly adjusted by those skilled in the art depending on the target sample, the target sequence to be detected, and the like. Examples of the preferred concentrations are final concentrations of 100 nM to 5 µM for common oligos, and 1 µM to 10 µM for query oligos, and the like.

### (Ligation)

The query oligo and the common oligo are subjected to a ligation reaction using a nucleic acid sample as a template, so that a ligation product to be hybridized to a solid-phase carrier may be obtained. The ligation as used herein is a step of connecting the query oligo and the common oligo bound to a corresponding nucleic acid sample. Since the query oligo and the common oligo bound to the nucleic acid sample are in a state of being adjacent to each other, it is possible to connect the query oligo and the common oligo via a connection part by the action of a ligase or the like to perform a ligation reaction. In the ligation reaction at this time, multiplex reactions that may simultaneously amplify a plurality of genes may be performed. The number of genes that may be amplified is 2 to 15. It is preferably 3 to 10, and further preferably 4 to 8.

Examples of suitable reagents that may be used to connect the oligos include, but are not limited thereto, Taq DNA Ligase from *Thermus aquaticus.* Also, both probe groups may be connected by a chemical method instead of the enzymatic method.

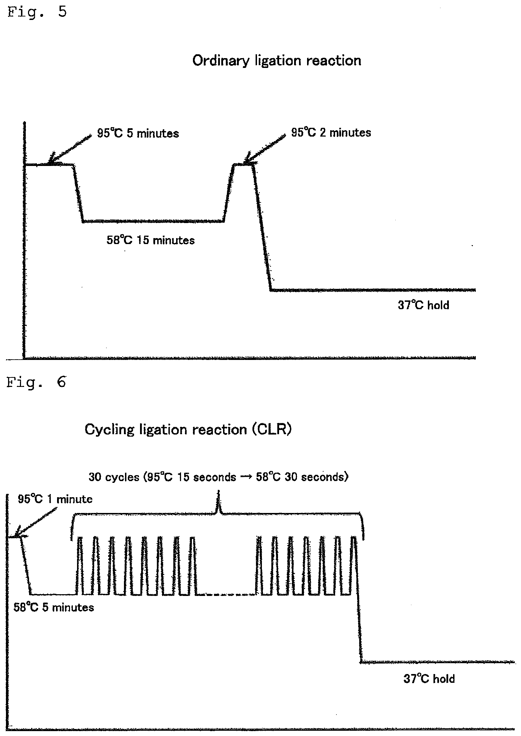

More specifically, as shown in Fig. 5, after bonding the nucleic acid sample with the query oligo and the common oligo, the query oligo and the common oligo are connected by a reaction at a high temperature of 90°C or more (for example, 90 to 100°C) for a few minutes, then at a temperature of about 50°C (for example, 40 to 60°C) for several tens of minutes, so that a determination of genetic polymorphism or the like may be performed with a strip (in this case, PAS is used). More specifically, the ligation treatment may be performed at a high temperature of 90°C or more for several minutes, then at a temperature of about 50°C for 10 to 20 minutes, further at a high temperature of 90°C or more for several minutes, and then at a lowering temperature of about 40°C. Preferably, the accuracy may be enhanced by further performing a cycling ligation reaction described below.

### (Cycling ligation reaction)

When LCR is used to obtain a large amount of ligation products, there is a problem of inspection accuracy as described above. Therefore, the present inventors have combined a heat treatment at 90°C or more with a ligation reaction at about 50°C, without adding complementary strands of the query oligo and the common oligo, so that a determination of genetic polymorphism or the like with high sensitivity and high accuracy may be performed (Fig. 6). This temperature adjustment method is a new ligation method different from a simple ligation reaction, and also different from LCR because it does not include complementary strands of the query oligo and the common oligo. The present inventors have named this temperature regulation method combining a heat treatment and a ligation reaction as a cycling ligation reaction (CLR). This reaction is herein called a "cycling ligation reaction".

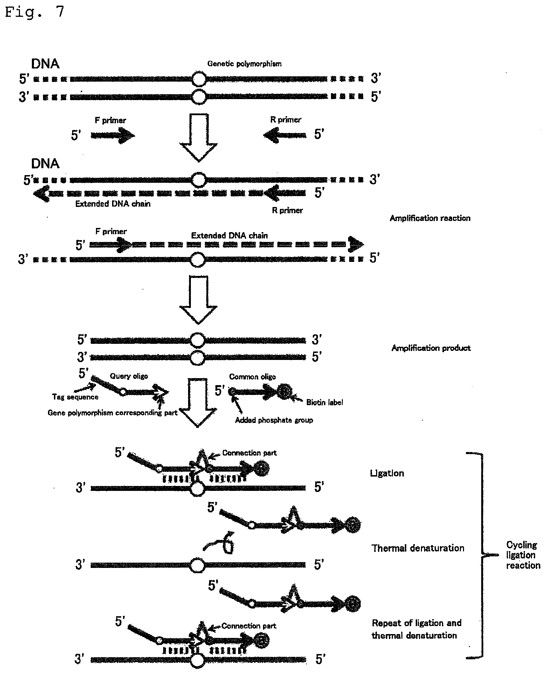

More specifically, as shown in Fig. 7, in the ligation in which the query oligo and the common oligo are hybridized to the nucleic acid sample, each of the time for hybridization and the time for ligation is set to a short time of about 10 to 30 seconds, and the temperature is set at a relatively high temperature. After the reaction, the step of rapidly raising the temperature, dissociating the double-stranded nucleic acid sample from the DNA product, lowering the temperature again, and performing the ligation reaction is performed in 20 or more cycles. This reaction is characterized by repeatedly performing the following four steps in a short time: 1. rapidly raising the temperature, 2. performing a high temperature treatment in a short time, 3 rapidly lowering the temperature, and 4. performing the ligation reaction in a short time. By this cycling ligation reaction, it is possible to amplify only the target single-stranded DNA accurately in a short time, and it enables to perform a detection with higher accuracy on a strip.

In the cycling ligation reaction, a high temperature of 90°C or more (for example, 90 to 100°C) and a temperature of about 50°C (for example, 40 to 60°C) are repeated (for example, 30 cycles of 90°C for 15 seconds and 58°C for 30 seconds). The preferred number of cycles of the cycling ligation reaction is, for example, 10 times or more, 20 times or more, or 30 times or more. The lower limit and upper limit of the number of cycles are not particularly limited, but the lower limit is, for example, 2 times or more, 3 times or more, 4 times or more, or 5 times or more, and the upper limit is, for example, 100 times or less, 90 times or less, 80 times or less, 70 times or less, 60 times or less, 50 times or less, or 40 times or less. The time of one cycle is not also particularly limited, and the lower limit is, for example, 10 seconds or more, 20 seconds or more, or 30 seconds or more, and the upper limit is 10 minutes or less, 5 minutes or less, 3 minutes or less, 2 minutes or less, 1 minute or less, or the like. The time for hybridization and the time for ligation may be the same or different from each other, and may be appropriately set according to the sample to be prepared or the like. The upper limit and lower limit of each of the time for hybridization and the time for ligation are not particularly limited, and examples of the lower limit include, for example, 10 seconds or more, 15 seconds or more, 30 seconds or more, 40 seconds or more, or 50 seconds or more, and examples of the upper limit include, for example, 5 minutes or less, 3 minutes or less, 2 minutes or less, 1 minute or less, 50 seconds or less, 40 seconds or less, or 30 seconds or less.

In the conventional method using a strip, it is necessary to use a spacer sequence between the single-stranded tag sequence and the sample for subjecting the amplified double-stranded DNA to detection. The method of the present invention enables to provide a simple and short-time genetic analysis since the single-stranded ligation product may be subjected to a hybridization treatment without PCR amplification, and thus a spacer sequence is unnecessary.

When the ligation treatment is performed, it is preferable to prepare a positive control that may be generated simultaneously with the nucleic acid sample. Examples of the positive control include G3PDH gene and the like. By performing detection of the sample and the gene product of the positive control at the same time, it enables to discriminate whether the ligation treatment causes a mistake or a corresponding sample is not contained.

### (Detection using a solid-phase carrier)

The ligation product obtained by the ligation tretment may be detected by subjecting to a solid-phase carrier on which a detection probe is immobilized. For example, in the case of using a chromatography main body such as a strip, the ligation product is added to a developing solution, and then a buffer, a color developing solution and the like are added thereto, if necessary, and mixed, and a hybridization reaction is performed by chromatography. The method of the present invention makes the result of genetic analysis visually detectable.

The "visually detectable" as used herein naturally includes a mode of visual detection, but it is not necessary to actually perform visual detection, and also includes, for example, a mode in which the result is read by an instrument such as a reader. Further, a state is also included in which visual inspection is possible by performing an additional operation, for example, light irradiation.

As mentioned in the above, the method of the present invention is used for various applications. Examples of the specific applications include, but are not limited to, genetic diagnosis of genetic diseases at medical sites, discrimination of the type of pathogen, quarantine at customs, inspection of varieties of meat and plants at food factories and food stores, determination of the presence or absence of use of a genetically modified raw material, and the like.

In other aspects of the present invention, the present invention relates to a kit that may be used in the method of genetic analysis.

In one embodiment, the kit of the present invention contains a strip provided with a detection probe, a query oligo containing a tag sequence hybridizable to a detection probe and a first single-stranded oligonucleotide, and a common oligo containing a second single-stranded oligonucleotide and a labeling moiety. In other embodiments, the kit of the present invention contains a strip provided with a detection probe, a query oligo containing a labeling moiety and a first single-stranded oligonucleotide, and a common oligo containing a second single-stranded oligonucleotide and a tag sequence hybridizable to the detection probe. In yet other embodiments, the kit of the present invention contains a solid-phase carrier provided with a detection probe, a query oligo containing a tag sequence hybridizable to the detection probe and a first single-stranded oligonucleotide, and a common oligo containing a second single-stranded oligonucleotide and a tag sequence hybridizable to a labeled probe.

The labeled probe as used herein refers to an oligonucleotide probe having a labeling moiety, which hybridizes to a tag sequence that does not hybridize to a detection probe, typically when both ends of the single-stranded DNA product have a tag sequence.

Preferably, the kit of the present invention further contains a ligase. Any ligase may be used as long as it is used for the ligation treatment. An example of a preferred ligase is a Taq DNA ligase.

The kit of the present invention may be used for analyzing sequence of any gene. For example, the kit may be used for a genetic analysis of bacteria, viruses, plants, animals including humans, and animals other than humans, and may be used for detection of gene mutations such as variations, genetic polymorphisms, and mutations, and the like. More specifically, the kit of the present invention may be used for, for example, detection and type determination of HPV, dengue fever, and influenza virus; determination of species of animals and plants; detection of genetically modified crops; halal certification inspection; and detection of viral drug resistant strain gene, drug resistant strain gene, single nucleotide polymorphism (SNP), microsatellite, substitution, deletion, insertion, and the like. For example, the kit of the present invention is used for detection of genetic polymorphisms, examination of food fraud of bovine, porcine or the like, and simple inspection of viral infection.

Specific examples will be described below, but the present invention is not necessarily limited thereto.

For the genetic test of humans of the present invention, it enables to easily perform a test by extracting genes from human blood and utilizing the method of the present invention. Examples of the gene include cytochrome genes, and the metabolic ability of drugs may be known. For example, it enables to know the ease of metabolism of nicotine in the body, the side effects of anticancer drugs such as irinotecan and tamoxifen, the effects of medicines, and the like.

For the genetic test of animals of the present invention, it enables to discriminate the species by using, for example, the method of the present invention for mitochondrial genes of various animals. Therefore, it is unnecessary to collect blood and the like while the animal is alive, and genes may be acquired even from meat for foods. Besides, processed products, raw materials for soup and the like may be used. The animal is not limited to bovine, porcine, and avian, and may be sheep, caprine, piscine, or the like.

For the test of pathogenic bacteria of the present invention, viruses or the like, it enables to easily perform the test by utilizing the method of the present invention for, for example, a sample collected directly from an infected person. The pathogenic bacteria and viruses are not particularly limited, and not only the presence or absence of infection with pathogenic bacteria or viruses but also the type discrimination may be performed.

The above test does not require any special device, and may be performed as long as a desktop device is available. It may be assumed to use the present invention in many situations, such as use in clinical sites including hospitals, inspection of processes at food factories, inspection of mislabeled food contamination at customs and the like, determination of parentage of animals, and determination of crop varieties.

Hereinafter, the present invention will be described in detail by way of examples, but the examples are for the purpose of illustration, and the present invention is not necessarily limited to the examples.

### Examples

### Example 1

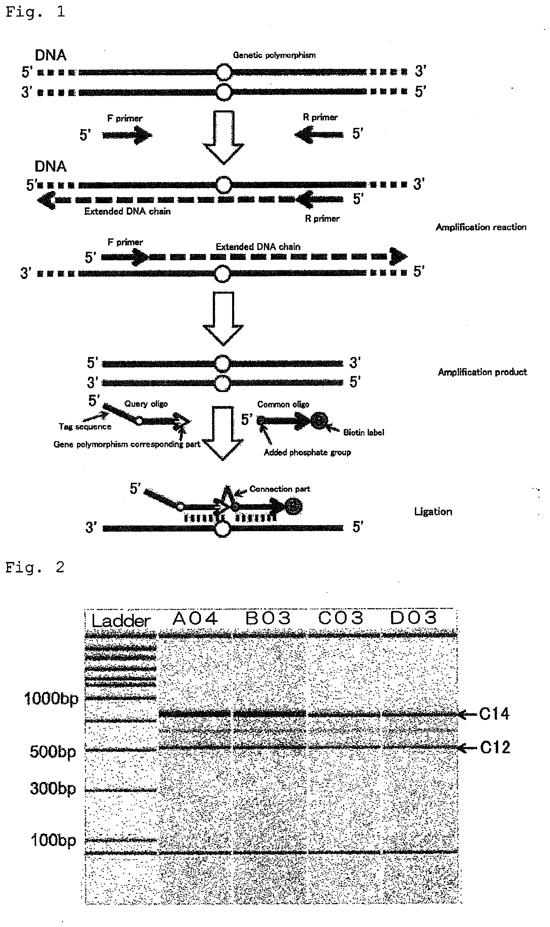

A detection of target gene mutation of the present invention was performed in accordance with the following procedures. The description will be given hereinbelow according to the following order. The procedure up to the ligation reaction is shown in Fig. 1.
1. Preparation of Primers
2. Amplification of Target Gene
3. Preparation of Ligation Probe
4. Ligation Reaction
5. Detection Using Chromatography Main Body (PAS: Printed Array Strip)
In addition, 4 samples of genomic DNAs (A04, B03, C03, D03) derived from human were subjected to experiments.

### 1. Preparation of Primers

Primers C12 and C14 (manufactured by Eurofins Genomics) specific to the CYP2D6 gene in the human genome shown in Table 1 below were prepared. These primers were synthesized in accordance with a usual oligonucleotide synthesis method, and adjusted to a concentration of 100 µM. The C12 primer was designed so as to amplify a gene region containing a genetic polymorphism of the same gene rs16947 (C mutated to T) and the C14 primer was designed so as to amplify a gene region containing a genetic polymorphism of the same genetic polymorphism rs1065852 (C mutated to T). In the figures, rs16947 and rs1065852 are described as 2850C>T and 100C>T, respectively.

**[Table 1]**

| Name | | Sequence (5' → 3') | SEQ ID NO. |
|---|---|---|---|
| C12 | F | GACCTGACTGAGGCCTTCCTGGCAGAGATGGAGAA | 1 |
| | R | TATGTTGGAGGAGGTCAGGCTTACAGGATCCTGGTCAA | 2 |
| C14 | F | TTGGCCTTTGGAAAATCCAGTCCTTCATGCCATGT | 3 |
| | R | CACTGAAACCCTGGTTATCCCAGAAGGCTTTGCAGGCTTCA | 4 |

### 2. Amplification of Target Gene

### 2-1. Preparation of Reaction Liquid

The genomic DNA used for amplification of the target gene was derived from human. The genomic DNA was amplified by using the primers in Table 1 as follows. As a reagent for sample amplification, Multiplex PCR Kit from QIAGEN was used. As a thermal cycler, Mastercycler (registered trademark) gradient from Eppendorf was used. The reagents shown in Table 2 were prepared for each individual sample.

**[Table 2]**

| Reagent name | Amount per sample |
|---|---|
| dH₂O | 3.5 µL |
| 2 × Multiplex PCR master mix | 5.0 µL |
| Primer mixtures (500 nM each) | 0.5 µL |
| Genomic DNA (25 ng/µL) | 1.0 µL |
| Total | 10 µL |

### 2-2. PCR Reaction

The prepared sample solution was transferred to a PCR tube, and a thermal cycle reaction (95°C for 15 minutes, followed by 40 cycles at 95°C for 30 seconds and 68°C for 2 minutes, and finally lowered to 4°C) was performed. Then, the amplified sample was subjected to electrophoresis using a bioanalyzer from Agilent Technologies, and it was confirmed that a target size amplification product was obtained. The results are shown in Fig. 2.

### 3. Preparation of Ligation Probe

### 3-1. Preparation of Ligation Probe

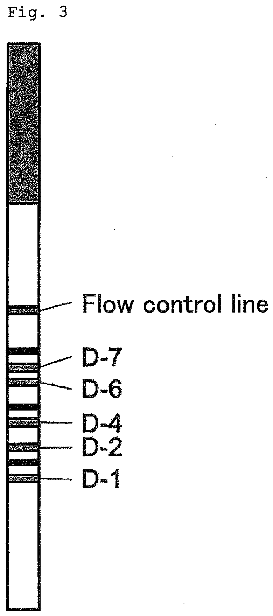

A ligation probe (common oligo manufactured by Eurofins Genomics, query oligo manufactured by TBA) to be bound to the amplified sample obtained in section 2. was prepared. The base sequences of the probes are shown in Table 3. For the ligation probe, one common oligo and two query oligos were prepared for each genetic polymorphism. The query oligo included one that binds to a wild type in which no genetic mutation has occurred (written as WT) and one that binds to a mutant type in which a genetic mutation has occurred (written as MT). Biotin [Biotin] is labeled at the 3'-end of the common oligo. A tag sequence complementary to the oligo DNA immobilized on the chromatography main body (PAS) is added at the 5'-end of the query oligo (sequence information not disclosed). Spacer C3 is inserted between the query oligo and the tag sequence. The position of the oligo DNA immobilized on the chromatography main body was shown in Fig. 3.

**[Table 3]**

| Target | Name | Type | Sequence (5' → 3') | SEQ ID NO. |
|---|---|---|---|---|
| rs16947 | C12-Com | Common oligo | CAGGTTCTCATCATTGAAGCTGCTCTCAGG [Biotin] | 5 |
| | C12-Que-WT | Query oligo | [D-6] -Spacer C3-AGCCACCACTAAGCG | 6 |
| | C12-Que-MT | Query oligo | [D-7] -Spacer C3-CAGCCACCACTAAGCA | 7 |
| rs1065852 | C14-Com | Common oligo | CACCAGGCCCCCTGCCACTG[Biotin] | 8 |
| | C14-Que-WT | Query oligo | [D-2] -Spacer C3-GGCTGCACGCAACC | 9 |
| | C14-Que-MT | Query oligo | [D-4] -Spacer C3-GGCTGCACGCAACT | 10 |

### 3-2. Phosphorylation at 5'-End of Common Oligo

In order to perform the ligation reaction, a phosphate group was added to the 5'-end of the common oligo. T4 Polynucleotide Kinase manufactured by Toyobo Co., Ltd. was used as a reagent for adding a phosphate group.

### 3-3. Preparation of Reaction Liquid

The reagents shown in Table 4 were prepared. In order to determine optimum concentration of common oligo, 4 concentrations, 1 µM, 10 µM, 50 µM, and 100 µM of common oligo were added to the prepared liquid.

**[Table 4]**

| Reagent name | Amount per reaction |
|---|---|
| dH₂O | 8.0 µL |
| 10 × Protruding End Kinase Buffer | 2.0 µL |
| Common oligo (1 µM, 10 µM, 50 µM, 100 µM) | 2.0 µL |
| γATP (manufactured by Toyobo Co., Ltd.) | 6.0 µL |
| T4 Polynucleotide Kinase | 2.0 µL |
| Total | 20 µL |

### 3-4. Reaction Conditions

A common oligo solution was transferred to a PCR tube and set in a thermal cycler. The temperature condition was at 37°C for 30 minutes, followed by 95°C for 3 minutes, then lowered to 4°C to finish the reaction.

### 3-5. Preparation of Reaction Liquid

To the total amount (20 µL) of the common oligo solution subjected to the phosphate group addition reaction, 20 µL of each of the query oligos (100 nM each) was mixed.

**[Table 5]**

| Common oligo liquid name | Concentration of query oligo added | Final concentration | | Solution No. |
|---|---|---|---|---|
| | | Common | Query | |
| Common oligo liquid 1 (containing each 1 µM, 2.0µL) | Each query oligo 100 nM | 50 nM | 50 nM | L1 |
| Common oligo liquid 2 (containing each 10 µM, 2.0µL) | Each query oligo 1 µM | 500 nM | 500 nM | L2 |
| | Each query oligo 5 µM | 500 nM | 2.5 µM | L3 |
| | Each query oligo 10 µM | 500 nM | 5 µM | L4 |
| Common oligo liquid 3 (containing each 50 µM, 2.0µL) | Each query oligo 5 µM | 2.5 µM | 2.5 µM | L5 |
| Common oligo liquid 4 (containing each 100 µM, 2.0µL) | Each query oligo 10 µM | 5 µM | 5 µM | L6 |

### 4. Ligation Reaction

### 4-1. Preparation of Reaction Liquid

The composition in the ligation reaction was as shown in Table 6. As an enzyme for the ligation, Taq DNA Ligase from New England Biolabs Japan Inc. was used.

**[Table 6]**

| Reagent name | Amount per reaction |
|---|---|
| dH₂O | 8.0 µL |
| 10 × Taq DNA ligase buffer | 1.5 µL |
| Oligo solutions (L1, L2, L3, L4, L5 or L6) | 2.0 µL |
| Taq DNA ligase | 0.5 µL |
| Amplified sample obtained in section 2. | 3.0 µL |
| Total | 15 µL |

### 4-2. Ligation Reaction

A ligation solution was transferred to a PCR tube and set in a thermal cycler. The temperature condition was at 95°C for 5 minutes, followed by 58°C for 15 minutes, 95°C for 2 minutes, then lowered to 37°C to finish the reaction.

### 5. Detection Using Chromatography Main Body

### 5-1. Hybridization Reaction

A hybridization reaction by nucleic acid chromatography with use of the single-stranded DNA obtained in section 4. and its detection were performed.

### 5-2. Preparation of Reaction Liquid

The hybridization reaction and the detection were performed by using a reaction solution prepared by mixing a developing solution (manufactured by TBA), a latex solution (manufactured by TBA), a TE buffer containing 30% formamide, and a ligation product. The composition of the reaction solution is shown in Table 7.

**[Table 7]**

| Reaction solution composition | |
|---|---|
| Reagent name | Amount per reaction |
| Developing solution (manufactured by TBA) | 20.0 µL |
| Latex solution (manufactured by TBA) | 2.0 µL |
| TE buffer (containing 30% formamide) | 10.0 µL |
| Ligation product | 10.0 µL |
| Total | 42.0 µL |

### 5-3. Hybridization Step

To a 1.5 mL tube, 50 µL of each of the above-mentioned reaction solutions was added, and each lower end part of the chromatography main body was inserted to perform a hybridization reaction by chromatography. The reaction solution was completely absorbed in about 20 minutes, and the hybridization reaction by chromatography was completed at the stage when all was absorbed. After completion of the reaction, the chromatography main body was air-dried, then the reaction site was visually confirmed, and the image was taken.

### 5-4. Detection Step

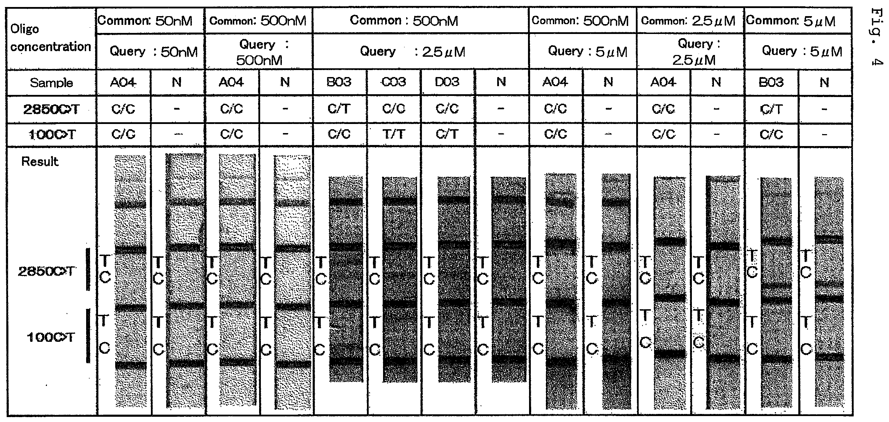

The presence or absence of coloring after drying the chromatography main body was visually confirmed. Results are shown in Table 8 and Fig. 4. As shown in Fig. 4, the solution having 500 nM of common oligo concentration and 2.5 µL of query oligo concentration prepared in section 3, Preparation of Ligation Probe provide color at a position to be expected. Thus, it was confirmed that the method is effective.

### 5-5. Results

**[Table 8]**

| Common oligo liquid name | Concentration of query oligo added | Final concentration | | Results |
|---|---|---|---|---|
| | | Common | Query | |
| Common oligo liquid 1 (containing each 1 µM, 2.0µL) | Each query oligo 100 nM | 50 nM | 50 nM | × |
| Common oligo liquid 2 (containing each 10 µM, 2.0µL) | Each query oligo 1 µM | 500 nM | 500 nM | × |
| | Each query oligo 5 µM | 500 nM | 2.5 µM | ○ |
| | Each query oligo 10 µM | 500 nM | 5 µM | × |
| Common oligo liquid 3 (containing each 50 µM, 2.0µL) | Each query oligo 5 µM | 2.5 µM | 2.5 µM | × |
| Common oligo liquid 4 (containing each 100 µM, 2.0µL) | Each query oligo 10 µM | 5 µM | 5 µM | × |

### Example 2

As a method for improving detection sensitivity of a target gene mutation of the present invention, experiments were performed by the following procedures. The description will be given hereinbelow according to the following order. The procedure up to the ligation reaction is shown in Fig. 7.
1. Preparation of Primers
2. Amplification of Target Gene
3. Preparation of Ligation Probe
4. Cycling Ligation Reaction
5. Detection Using Chromatography Main Body (PAS: Printed Array Strip)

In addition, genomic DNAs (A, B, C, D, E, F, and G) derived from human were subjected to experiments.

### 1. Preparation of Primers

Primers C09, C10, C12 and C14 specific to CYP2D6 gene in the human genome and shown in Table 9 below and a primer control specific to G3PDH gene (manufactured by Eurofins Genomics) as a positive control were prepared. These primers were synthesized in accordance with a usual oligonucleotide synthesis method, and adjusted to a concentration of 100 µM. The C09 primer was designed so as to amplify a gene region containing a genetic polymorphism of the CYP2D6 gene rs1135840 (G mutated to C). The C10 primer was designed so as to amplify a gene region containing a genetic polymorphism of the same gene rs5030865 (G mutated to A). The C12 primer was designed so as to amplify a gene region containing a genetic polymorphism of the same gene rs16947 (C mutated to T). And, the C14 primer was designed so as to amplify a gene region containing a genetic polymorphism of the same genetic polymorphism rs1065852 (C mutated to T). In the figures, rs1135840, rs5030865, rs16947 and rs1065852 are described as 4180G>C, 1758G>A, 2850C>T and 100C>T, respectively.

**[Table 9]**

| Name | | Sequence (5' → 3') | SEQ ID NO. |
|---|---|---|---|
| C09 | F | CTGACATCTGCTCAGCCGCAACGTACCCCTG | 11 |
| | R | ACCTGCTGCAGCACTTCAGCTTCTCGGTGCCCACT | 12 |
| C10 | F | AAGAAACCACCTGCACTAGGGAGGTGTGAGCATGGG | 13 |
| | R | AAACCCATCTATGCAAATCCTGCTCTTCCGAGGCC | 14 |
| C12 | F | GACCTGACTGAGGCCTTCCTGGCAGAGATGGAGAA | 1 |
| | R | TATGTTGGAGGAGGTCAGGCTTACAGGATCCTGGTCAA | 2 |
| C14 | F | TTGGCCTTTGGAAAATCCAGTCCTTCATGCCATGT | 3 |
| | R | CACTGAAACCCTGGTTATCCCAGAAGGCTTTGCAGGCTTCA | 4 |
| Control | F | GGAATGGGACTGAGGCTCCCACCTTTCTCAT | 15 |
| | R | GCGTCAAAGGTGGAGGAGTGGGTGTCG | 16 |

### 2. Amplification of Target Gene

### 2-1. Preparation of Reaction Liquid

The genomic DNA used for amplification of the target gene was derived from human. Genomic DNA was amplified as follows using the primers in Table 9. Here, as a reagent for sample amplification, KAPA2G FAST Multiplex from KAPA BIOSYSTEMS was used. As a thermal cycler, Mastercycler (registered trademark) gradient from Eppendorf was used. The reagents shown in Table 10 were prepared for each individual sample.

**[Table 10]**

| Reagent name | Amount per sample |
|---|---|
| dH₂O | 3.5 µL |
| 2 × KAPA2G FAST Multiplex | 5.0 µL |
| Primer mixtures (500 nM each) | 0.5 µL |
| Genomic DNA (25 ng/µL) | 1.0 µL |
| Total | 10 µL |

### 2-2. PCR Reaction

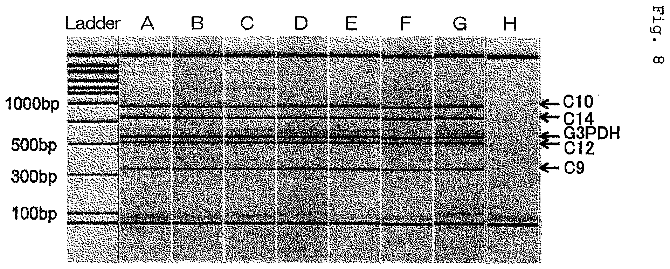

The prepared sample solution was transferred to a PCR tube, and a thermal cycle reaction (95°C for 3 minutes, followed by 30 cycles at 95°C for 15 seconds, 60°C for 30 seconds and 72°C for 1 minute, then 72°C for 5 minutes, and finally lowered to 4°C) was performed. Then, the amplified sample was subjected to electrophoresis using a bioanalyzer from Agilent Technologies, and it was confirmed that a target size amplification product was obtained. The results are shown in Fig. 8.

### 3. Preparation of Ligation Probe

### 3-1. Preparation of Ligation Probe

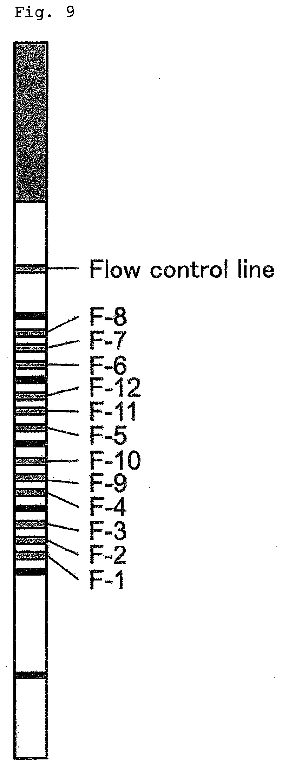

A ligation probe (common oligo manufactured by Eurofins Genomics, query oligo manufactured by TBA) to be bound to the amplified sample obtained in section 2. was prepared. The base sequences of the probes are shown in Table 11. For the ligation probe, one common oligo and two query oligos were prepared for each genetic polymorphism. The query oligo included one that binds to a wild type in which no genetic mutation has occurred (written as WT) and one that binds to a mutant type in which a genetic mutation has occurred (written as MT). A phosphate group [P] is modified at the 5'-end of the common oligo, and biotin [Biotin] is labeled at the 3'-end of the common oligo. A tag sequence complementary to the oligo DNA immobilized on the chromatography main body (PAS) is added at the 5'-end of the query oligo (sequence information not disclosed). Spacer C3 is inserted between the query oligo and the tag sequence. The position of the oligo DNA immobilized on the chromatography main body was shown in Fig. 9. In the present example, the E12 series of chromatography main body sold by TBA was used.

**[Table 11]**

| Target | Name | Type | Sequence (5' → 3') | SEQ ID NO. |
|---|---|---|---|---|
| rs1135840 | C09-Com | Common oligo | [P] TCACCAGGAAAGCAAAGACACCATGGGCATC TTGTATGAAGTACCCCCC[Biotin] | 17 |
| | C09-Que-WT | Query oligo | [F-1] -Spacer C3-CATAGGGGGATGGGC | 18 |
| | C09-Que-MT | Query oligo | [F-2] -Spacer C3-TCATAGGGGGATGGGG | 19 |
| rs5030865 | C10-Com | Common oligo | [P] GTGGGTGATGGGCAGAAGGGCTCTAGGGTAT TTGCCTGCTACTC[Biotin] | 20 |
| | C10-Que-WT | Query oligo | [F-3] -Spacer C3-CGCCAACCACACCG | 21 |
| | C10-Que-MT | Query oligo | [F-4] -Spacer C3-CGCCAACCACACCA | 1622 |
| rs16947 | C12-Com | Common oligo | [P] CAGGTTCTCATCATTGAAGCTGCTCTCAGG [Biotin] | 5 |
| | C12-Que-WT | Query oligo | [F-5] -Spacer C3-AGCCACCACTAAGCG | 6 |
| | C12-Que-MT | Query oligo | [F-6] -Spacer C3-CAGCCACCACTAAGCA | 7 |
| rs1065852 | C14-Com | Common oligo | [P] CACCAGGCCCCCTGCCACTG[Biotin] | 8 |
| | C14-Que-WT | Query oligo | [F-7] -Spacer C3-GGCTGCACGCAACC | 9 |
| | C14-Que-MT | Query oligo | [F-8] -Spacer C3-GGCTGCACGCAACT | 10 |
| G3PDH | G3-Com | Common oligo | [P] AAGGTCATCCCTGAGCTGAACG[Biotin] | 23 |
| | G3-Que | Query oligo | [F-9] -Spacer C3-GGAAGCTCACTGGCATGGCCTTCCGT | 24 |

### 4. Cycling Ligation Reaction

### 4-1. Preparation of Reaction Liquid

The composition in the ligation reaction was as shown in Table 12. As an enzyme for the ligation, Taq DNA Ligase from New England Biolabs Japan Inc. was used.

**[Table 12]**

| Reagent name | Amount per reaction |
|---|---|
| dH₂O | 8.0 µL |
| 10 × Taq DNA ligase buffer | 1.5 µL |
| Common oligo mixtures (1 µM each) | 1.0 µL |
| Query oligo mixtures (5 µM each) | 1.0 µL |
| Taq DNA ligase | 0.5 µL |
| Amplified sample obtained in section 2. | 3.0 µL |
| Total | 15 µL |

### 4-2. Ligation Reaction

A ligation solution was transferred to a PCR tube and set in a thermal cycler. The temperature condition was at 95°C for 1 minute, followed by 58°C for 5 minutes, then 30 cycles at 95°C for 15 seconds and 58°C for 30 seconds, then at 95°C for 1 minute, and the temperature was lowered to 37°C to finish the reaction.

### 5. Detection Using Chromatography Main Body

### 5-1. Hybridization Reaction

A hybridization reaction by nucleic acid chromatography with use of the single-stranded DNA obtained in section 4. and its detection were performed.

### 5-2. Preparation of Reaction Liquid

The hybridization reaction and the detection were performed by using a reaction solution prepared by mixing a developing solution (manufactured by TBA), a latex solution (manufactured by TBA), a TE buffer, and a ligation product. The composition of the reaction solution is shown in Table 13.

**[Table 13]**

| Reaction solution composition | |
|---|---|
| Reagent name | Amount per reaction |
| Developing solution (manufactured by TBA) | 10.0 µL |
| Latex solution (manufactured by TBA) | 2.0 µL |
| TE buffer | 3.0 µL |
| Ligation product | 5.0 µL |
| Total | 20.0 µL |

### 5-3. Hybridization Step

To a 1.5 mL tube, 20 µL of each of the above-mentioned reaction solutions was added, and each lower end part of the chromatography main body was inserted to perform a hybridization reaction by chromatography. The hybridization reaction was performed at 37°C. The reaction solution was completely absorbed in about 20 minutes, and the hybridization reaction by chromatography was completed at the stage when all was absorbed. After completion of the reaction, the chromatography main body was air-dried, then the reaction site was visually confirmed, and the image was taken.

### 5-4. Detection Step

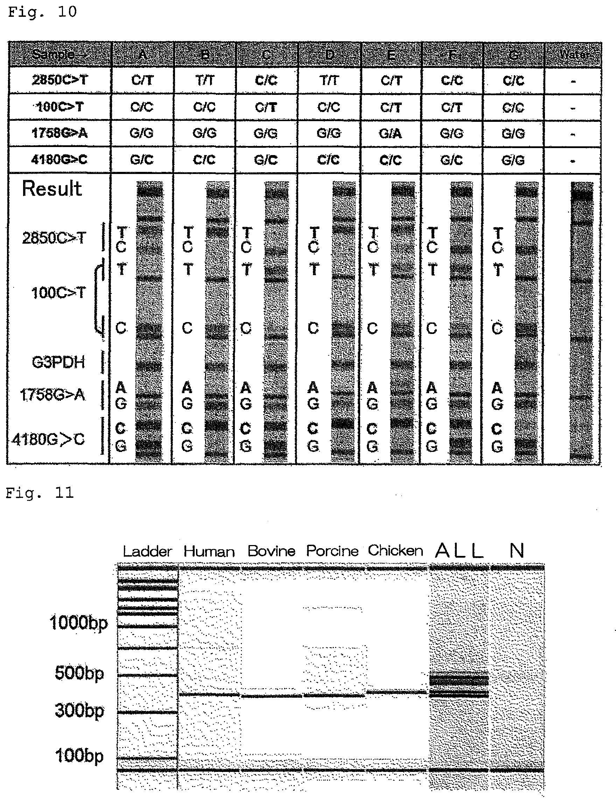

The presence or absence of coloring after drying the chromatography main body was visually confirmed. As shown in Fig. 10, known genetic polymorphisms are all matched with the analysis results of genetic polymorphisms by the chromatography main body. The cycling ligation reaction enabled the line on the chromatography main body to be more clearly visible than Example 1. Accordingly, it was shown that the method of the present invention may be used for a simple method for determining genetic polymorphism.

### Example 3

In order to demonstrate that the detection method of the present invention may be used not only for the method for determining genetic polymorphisms of human genes but also animal species, experiments were performed by the following procedures.
1. Preparation of Primers
2. Amplification of Target Gene
3. Preparation of Ligation Probe
4. Cycling Ligation Reaction
5. Detection Using Chromatography Main Body (PAS: Printed Array Strip)

In addition, genomic DNA derived from human, genomic DNA derived from bovine, genomic DNA derived from porcine, genomic DNA derived from chicken, and DNA obtained by mixing DNAs derived from bovine, porcine and chicken at 1:1:1 were subjected to experiments.

### 1. Preparation of Primers

Among mitochondrial DNAs in human, bovine, porcine and chicken genomes, a DNA sequence common to these four species was found, and the primers shown in Table 14 (manufactured by Eurofins Genomics) were prepared. These primers were synthesized in accordance with a usual oligonucleotide synthesis method, and adjusted to a concentration of 100 µM.

**[Table 14]**

| Name | | Sequence (5' → 3') | SEQ ID NO. |
|---|---|---|---|
| Common primer | F | AATAAGGACTTGTATGAATGGC | 25 |
| | R | CCAACATCGAGGTCGTAAACC | 26 |

### 2. Amplification of Target Gene

### 2-1. Preparation of Reaction Liquid

The genomic DNAs used for amplification of the target gene were derived from human, bovine, porcine, and chicken. The genomic DNAs were amplified using the primers in Table 6 as follows. As a reagent for sample amplification, KAPA2G FAST Multiplex from KAPA BIOSYSTEMS was used. As a thermal cycler, Mastercycler (registered trademark) gradient from Eppendorf was used. The reagents shown in Table 15 were prepared for each individual sample.

**[Table 15]**

| Reagent name | Amount per sample |
|---|---|
| dH₂O | 3.8 µL |
| 2 × KAPA2G FAST Multiplex | 5.0 µL |
| Primer mixtures (5 µM each) | 0.2 µL |
| Genomic DNA (10 ng/µL) | 1.0 µL |
| Total | 10 µL |

### 2-2. PCR Reaction

The prepared sample solution was transferred to a PCR tube, and a thermal cycle reaction (95°C for 3 minutes, followed by 30 cycles at 95°C for 15 seconds, 57°C for 30 seconds and 72°C for 1 minute, then 72°C for 5 minutes, and finally lowered to 4°C) was performed. Then, the amplified sample was subjected to electrophoresis using a bioanalyzer from Agilent Technologies, and it was confirmed that a target size amplification product was obtained. The results are shown in Fig. 11.

### 3. Preparation of Ligation Probe

### 3-1. Preparation of Ligation Probe

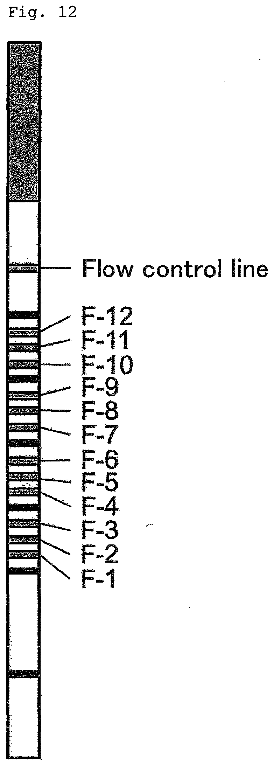

A ligation probe (common oligo manufactured by Eurofins Genomics, query oligo manufactured by TBA) to be bound to the amplified sample obtained in section 2. was prepared. The base sequences of the probes are shown in Table 16. For the ligation probe, one common oligo and one query oligo were prepared for each animal species. In addition, oligos and query oligos common to human, bovine, porcine and chicken were prepared for positive controls. A phosphate group [P] was modified at the 5'-end of the common oligo, and biotin [Biotin] was labeled at the 3'-end of the common oligo. A tag sequence complementary to the oligo DNA immobilized on the chromatography main body (PAS) was added at the 5'-end of the query oligo (sequence information not disclosed). In addition, in the present example, Spacer C3 was not inserted between the query oligo and the tag sequence. The position of the oligo DNA immobilized on the chromatography main body was shown in Fig. 12. In the present example, the F12 series of chromatography main body sold by TBA was used. The position of the immobilized oligo DNA is somewhat different from that of the E12 series used in Example 2.

**[Table 16]**

| Target | Name | Type | Sequence (5' → 3') | SEQ ID NO. |
|---|---|---|---|---|
| Human | Hum-Common | Common oligo | [P] CCACAGGTCCTAAACTACCAAACCTGC [Biotin] | 27 |
| | Hum-Query | Query oligo | [F-1] AATGCAAACAGTACCTAACAAAC | 28 |
| Bovine | Ush-Common | Common oligo | [P] ATTTAACCATTAAGGAATAACAACAATCTCC [Biotin] | 29 |
| | Ush-Query | Query oligo | [F-2] ACTAACCAACCCAAAGAGAATAG | 30 |
| Porcine | But-Common | Common oligo | [P] AACTCAACCACAAAGGGATAAAACATAACTTAAC [Biotin] | 31 |
| | But-Query | Query oligo | [F-3] CTTTAATTAACTATTCCAAAAGTTAAAC | 32 |
| Chicken | Niw-Common | Common oligo | [P] AACCTTACACAGCCCCACTGGGTCCACCC [Biotin] | 33 |
| | Niw-Query | Query oligo | [F-4] CTTTAAAATCACGACCACCTTAC | 34 |
| Common | Cont-Common | Common oligo | [P] ATAACAGCGCAATC[Biotin] | 35 |
| | Cont-Query | Query oligo | [F-9] TACCCYAGGG | 36 |

### 4. Cycling Ligation Reaction

### 4-1. Preparation of Reaction Liquid

The composition in the ligation reaction was as shown in Table 17. As an enzyme for the ligation, Taq DNA Ligase from New England Biolabs Japan Inc. was used.

**[Table 17]**

| Reagent name | Amount per reaction |
|---|---|
| dH₂O | 8.0 µL |
| 10 × Taq DNA ligase buffer | 1.5 µL |
| Common oligo mixtures (1 µM each) | 1.0 µL |
| Query oligo mixtures (5 µM each) | 1.0 µL |
| Taq DNA ligase | 0.5 µL |
| Amplified sample obtained in section 2. | 3.0 µL |
| Total | 15 µL |

### 4-2. Ligation Reaction

A ligation solution was transferred to a PCR tube and set in a thermal cycler. The temperature condition was at 95°C for 1 minute, followed by 58°C for 5 minutes, then 30 cycles at 95°C for 15 seconds and 58°C for 30 seconds, then at 95°C for 1 minute, and the temperature was lowered to 37°C to finish the reaction.

### 5. Detection Using Chromatography Main Body

### 5-1. Hybridization Reaction

A hybridization reaction by nucleic acid chromatography with use of the single-stranded DNA obtained in section 4. and its detection were performed.

### 5-2. Preparation of Reaction Liquid

The hybridization reaction and the detection were performed by using a reaction solution prepared by mixing a developing solution (manufactured by TBA), a latex solution (manufactured by TBA), a TE buffer and a ligation product. The composition of the reaction solution is shown in Table 18.

**[Table 18]**

| Reaction solution composition | |
|---|---|
| Reagent name | Amount per reaction |
| Developing solution (manufactured by TBA) | 10.0 µL |
| Latex solution (manufactured by TBA) | 2.0 µL |
| TE buffer | 3.0 µL |
| Ligation product | 5.0 µL |
| Total | 12.0 µL |

### 5-3. Hybridization Step

To a 1.5 mL tube, 20 µL of each of the above-mentioned reaction solutions was added, and each lower end part of the chromatography main body was inserted to perform a hybridization reaction by chromatography. The hybridization reaction was performed at 37°C. The reaction solution was completely absorbed in about 20 minutes, and the hybridization reaction by chromatography was completed at the stage when all was absorbed. After completion of the reaction, the chromatography main body was air-dried, then the reaction site was visually confirmed, and the image was taken.

### 5-4. Detection Step

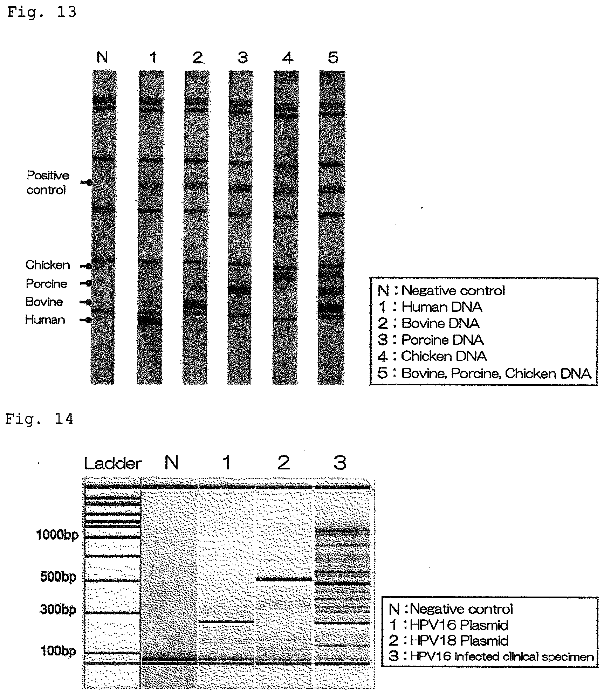

The presence or absence of coloring after drying the chromatography main body was visually confirmed. As shown in Fig. 13, each animal species determination by the chromatography main body was performed by the present method. Accordingly, it was shown that the method of the present invention may also be used for simple animal species determination.

### Example 4

In order to demonstrate that the detection method of the present invention may be used not only for the method for determining genetic polymorphisms of human genes but also the type of viruses and the like, experiments were performed by the following procedures. The detection targets were human papillomavirus types 16 and 18.
1. Preparation of Primers
2. Amplification of Target Gene
3. Preparation of Ligation Probe
4. Cycling Ligation Reaction
5. Detection Using Chromatography Main Body (PAS: Printed Array Strip)

As experimental materials, a plasmid into which a gene fragment of HPV16 and a gene fragment of HPV18 were introduced, and one sample of a clinical specimen known to be infected with HPV16 were used.

### 1. Preparation of Primers

Primers specific to HPV types 16 and 18 shown in Table 19 and a primer specific to human G3PDH gene (manufactured by Eurofins Genomics) as a positive control were prepared. These primers were synthesized in accordance with a usual oligonucleotide synthesis method, and adjusted to a concentration of 100 µM.

**[Table 19]**

| Name | | Sequence (5' → 3') | SEQ ID NO. |
|---|---|---|---|
| HPV16 | F | CGGTTGCATGCTTTTTGG | 37 |
| | R | CAGCGGTATGTAAGGCGTTG | 38 |
| HPV18 | F | CTGCACCGGCTGAAAATAAG | 39 |
| | R | ATAGCCCAACAAGCAACACC | 40 |
| Control | F | GGAATGGGACTGAGGCTCCCACCTTTCTCAT | 15 |
| | R | GCGTCAAAGGTGGAGGAGTGGGTGTCG | 16 |

### 2. Amplification of Target Gene

### 2-1. Preparation of Reaction Liquid

A plasmid containing a DNA sequence derived from HPV type 16 and a plasmid containing a DNA sequence derived from type 18 were used as the DNA used for amplification of the target gene. These plasmids contain the primer sequences of HPV16 and HPV18 shown in Table 19. In order to evaluate the performance of HPV type determination, human genomic DNA known to be infected with HPV16 was used for experiments. These DNAs were amplified by using the primers in Table 19 as follows. As a reagent for sample amplification, KAPA2G FAST Multiplex from KAPA BIOSYSTEMS was used. As a thermal cycler, Mastercycler (registered trademark) gradient from Eppendorf was used. The reagents shown in Table 20 were prepared for each individual sample.

**[Table 20]**

| Reagent name | Amount per sample |
|---|---|
| dH₂O | 3.8 µL |
| 2 × KAPA2G FAST Multiplex | 5.0 µL |
| Primer mixtures (5 µM each) | 0.2 µL |
| Genomic DNA (10 ng/µL) | 1.0 µL |
| Total | 10 µL |

### 2-2. PCR Reaction

The prepared sample solution was transferred to a PCR tube, and a thermal cycle reaction (95°C for 3 minutes, followed by 30 cycles at 95°C for 15 seconds, 57°C for 30 seconds and 72°C for 1 minute, then 72°C for 5 minutes, and finally lowered to 4°C) was performed. Then, the amplified sample was subjected to electrophoresis using a bioanalyzer from Agilent Technologies, and it was confirmed that a target size amplification product was obtained. The results are shown in Fig. 14.

### 3. Preparation of Ligation Probe

### 3-1. Preparation of Ligation Probe

A ligation probe (common oligo manufactured by Eurofins Genomics, query oligo manufactured by TBA) to be bound to the amplified sample obtained in section 2. was prepared. The base sequences of the probes are shown in Table 21. For the ligation probe, one common oligo and one query oligo were each prepared for HPV16, HPV18, and positive control human G3PDH gene. A phosphate group [P] was modified at the 5'-end of the common oligo, and biotin [Biotin] was labeled at the 3'-end of the common oligo. A tag sequence complementary to the oligo DNA immobilized on the chromatography main body (PAS) was added at the 5'-end of the query oligo (sequence information not disclosed). In addition, in the present example, Spacer C3 was not inserted between the query oligo and the tag sequence. The position of the oligo DNA immobilized on the chromatography main body was shown in Fig. 12. In the present example, the F12 series of chromatography main body sold by TBA was used. The position of the immobilized oligo DNA is somewhat different from that of the E12 series used in Example 2.

**[Table 21]**

| Target | Name | Type | Sequence (5' → 3') | SEQ ID NO. |
|---|---|---|---|---|
| HPV16 | Common-16 | Common oligo | [P] TGCCAAATCCCTGTTTTCCTGACC[Biotin] | 41 |
| | Query-16 | Query oligo | [F-1] CGTTTCCTGCTTGCCATGCG | 42 |
| HPV18 | Common-18 | Common oligo | [P] TAGGCCCTCGCAAACGTTCTGCT[Biotin] | 43 |
| | Query-18 | Query oligo | [F-2] ATTGCGTCGCAAGCCCACCA | 44 |
| G3PDH | G3-Com | Common oligo | [P] AAGGTCATCCCTGAGCTGAACG[Biotin] | 23 |
| | G3-Que | Query oligo | [F-9] -GGAAGCTCACTGGCATGGCCTTCCGT | 24 |

### 4. Cycling Ligation Reaction

### 4-1. Preparation of Reaction Liquid

The composition in the ligation reaction was as shown in Table 22. As an enzyme for the ligation, Taq DNA Ligase from New England Biolabs Japan Inc. was used.

**[Table 22]**

| Reagent name | Amount per reaction |
|---|---|
| dH₂O | 8.0 µL |
| 10 x Taq DNA ligase buffer | 1.5 µL |
| Common oligo mixtures (5 µM each) | 1.0 µL |
| Query oligo mixtures (5 µM each) | 1.0 µL |
| Taq DNA ligase | 0.5 µL |
| Amplified sample obtained in section 2. | 3.0 µL |
| Total | 15 µL |

### 4-2. Ligation Reaction

A ligation solution was transferred to a PCR tube and set in a thermal cycler. The temperature condition was at 95°C for 1 minute, followed by 58°C for 5 minutes, then 30 cycles at 95°C for 15 seconds and 58°C for 30 seconds, then at 95°C for 1 minute, and the temperature was lowered to 37°C to finish the reaction.

### 5. Detection Using Chromatography Main Body

### 5-1. Hybridization Reaction

A hybridization reaction by nucleic acid chromatography with use of the single-stranded DNA obtained in section 4. and its detection were performed.

### 5-2. Preparation of Reaction Liquid

The hybridization reaction and the detection were performed by using a reaction solution prepared by mixing a developing solution (manufactured by TBA), a latex solution (manufactured by TBA), a TE buffer and a ligation product. The composition of the reaction solution is shown in Table 23.

**[Table 23]**

| Reaction solution composition | |
|---|---|
| Reagent name | Amount per reaction |
| Developing solution (manufactured by TBA) | 10.0 µL |
| Latex solution (manufactured by TBA) | 2.0 µL |
| TE buffer | 3.0 µL |
| Ligation product | 5.0 µL |
| Total | 20.0 µL |

### 5-3. Hybridization Step

To a 1.5 mL tube, 20 µL of each of the above-mentioned reaction solutions was added, and each lower end part of the chromatography main body was inserted to perform a hybridization reaction by chromatography. The hybridization reaction was performed at 37°C. The reaction solution was completely absorbed in about 20 minutes, and the hybridization reaction by chromatography was completed at the stage when all was absorbed. After completion of the reaction, the chromatography main body was air-dried, then the reaction site was visually confirmed, and the image was taken.

### 5-4. Detection Step

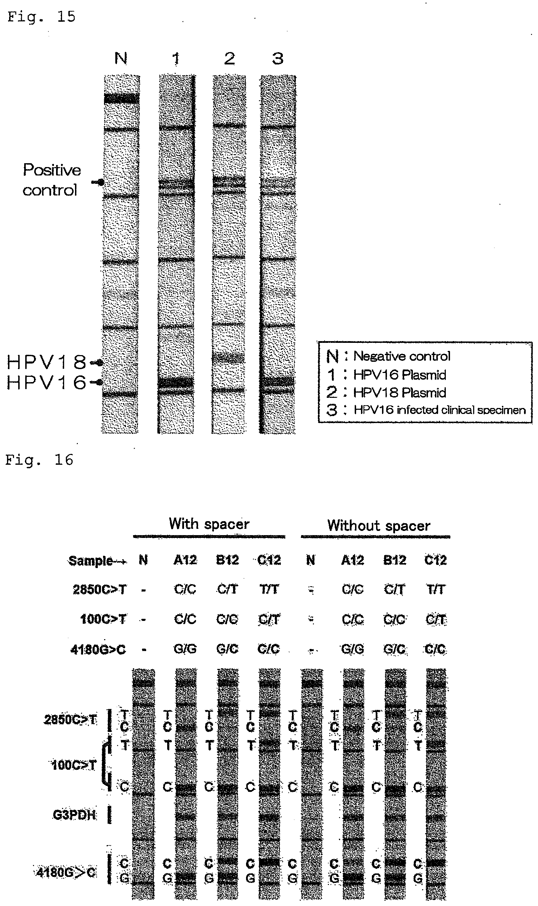

The presence or absence of coloring after drying the chromatography main body was visually confirmed. As shown in Fig. 15, it enabled to determine HPV types 16 and 18 by the chromatography main body according to the present method. Accordingly, it was shown that the present method is effective as a simple HPV type determination method.

### Example 5

In order to investigate whether the presence or absence of the spacer of the query tag influences the result of the test, experiments were performed by the following procedures. The description will be given hereinbelow according to the following order.
1. Preparation of Primers
2. Amplification of Target Gene
3. Preparation of Ligation Probe (no spacer)
4. Cycling Ligation Reaction
5. Detection Using Chromatography Main Body (PAS: Printed Array Strip)
Three samples of genomic DNAs (A12, B12, C12) derived from human were subjected to experiments.

### 1. Preparation of Primers

Primers C09, C10, C12 and C14 specific to the CYP2D6 gene in the human genome shown in Table 24 below and a primer control specific to the G3PDH gene (manufactured by Eurofins Genomics) as a positive control were prepared. These primers were synthesized in accordance with a usual oligonucleotide synthesis method, and adjusted to a concentration of 100 µM. The C09 primer was designed so as to amplify a gene region containing a genetic polymorphism of the CYP2D6 gene rs113584 (G mutated to C). The C12 primer was designed so as to amplify a gene region containing a genetic polymorphism of the same gene rs16947 (C mutated to T). And, the C14 primer was designed so as to amplify a gene region containing a genetic polymorphism of the same genetic polymorphism rs1065852 (C mutated to T). In the figures of examples, rs1135840, rs16947 and rs1065852 are described as 4180G>C, 2850C>T and 100C>T, respectively.

**[Table 24]**

| Name | | Sequence (5' → 3') | SEQ ID NO. |
|---|---|---|---|
| C09 | F | CTGACATCTGCTCAGCCGCAACGTACCCCTG | 11 |
| | R | ACCTGCTGCAGCACTTCAGCTTCTCGGTGCCCACT | 12 |
| C12 | F | GACCTGACTGAGGCCTTCCTGGCAGAGATGGAGAA | 1 |
| | R | TATGTTGGAGGAGGTCAGGCTTACAGGATCCTGGTCAA | 2 |
| C14 | F | TTGGCCTTTGGAAAATCCAGTCCTTCATGCCATGT | 3 |
| | R | CACTGAAACCCTGGTTATCCCAGAAGGCTTTGCAGGCTTCA | 4 |
| Control | F | GGAATGGGACTGAGGCTCCCACCTTTCTCAT | 15 |
| | R | GCGTCAAAGGTGGAGGAGTGGGTGTCG | 16 |

### 2. Amplification of Target Gene

### 2-1. Preparation of Reaction Liquid

The genomic DNA used for amplification of the target gene was derived from human. The genomic DNA was amplified by using the primers in Table 24 as follows. As a reagent for sample amplification, KAPA2G FAST Multiplex from KAPA BIOSYSTEMS was used. As a thermal cycler, Mastercycler (registered trademark) gradient from Eppendorf was used. The reagents shown in Table 25 were prepared for each individual sample.

**[Table 25]**

| Reagent name | Amount per sample |
|---|---|
| dH₂O | 3.5 µL |
| 2 × KAPA2G FAST Multiplex | 5.0 µL |
| Primer mixtures (500 nM each) | 0.5 µL |
| Genomic DNA (25 ng/µL) | 1.0 µL |
| Total | 10 µL |

2-2. PCR Reaction

The prepared sample solution was transferred to a PCR tube, and a thermal cycle reaction (95°C for 3 minutes, followed by 30 cycles at 95°C for 15 seconds, 60°C for 30 seconds and 72°C for 1 minute, then 72°C for 5 minutes, and finally lowered to 4°C) was performed.

### 3. Preparation of Ligation Probe

### 3-1. Preparation of Ligation Probe

A ligation probe (common oligo manufactured by Eurofins Genomics, query oligo manufactured by TBA) to be bound to the amplified sample obtained in section 2. was prepared. The base sequences of the probes are shown in Table 26. For the ligation probe, one common oligo and two query oligos were prepared for each genetic polymorphism. The query oligo included one that binds to a wild type in which no genetic mutation has occurred (written as WT) and one that binds to a mutant type in which a genetic mutation has occurred (written as MT). A phosphate group [P] was modified at the 5'-end of the common oligo, and biotin [Biotin] was labeled at the 3'-end of the common oligo. A tag sequence complementary to the oligo DNA immobilized on the chromatography main body (PAS) was added at the 5'-end of the query oligo (sequence information not disclosed). Spacer C3 was not inserted between the query oligo and the tag sequence. The position of the oligo DNA immobilized on the chromatography main body was shown in Fig. 9. In the present example, the E12 series of chromatography main body sold by TBA was used.

**[Table 26]**

| Target | Name | Type | Sequence (5' → 3') | SEQ ID NO. |
|---|---|---|---|---|
| rs1135840 | C09-Com | Common oligo | [P] TCACCAGGAAAGCAAAGACACCATGGGCATCTT GTATGAAGTACCCCCC[Biotin] | 17 |
| | C09-Que-WT | Query oligo | [F-1] CATAGGGGGATGGGC | 18 |
| | C09-Que-MT | Query oligo | [F-2] TCATAGGGGGATGGGG | 19 |
| rs16947 | C12-Com | Common oligo | [P] CAGGTTCTCATCATTGAAGCTGCTCTCAGG [Biotin] | 5 |
| | C12-Que-WT | Query oligo | [F-5] AGCCACCACTAAGCG | 6 |
| | C12-Que-MT | Query oligo | [F-6] CAGCCACCACTAAGCA | 7 |
| rs1065852 | C14-Com | Common oligo | [P] CACCAGGCCCCCTGCCACTG[Biotin] | 8 |
| | C14-Que-WT | Query oligo | [F-7] GGCTGCACGCAACC | 9 |
| | C14-Que-MT | Query oligo | [F-8] GGCTGCACGCAACT | 10 |
| G3PDH | G3-Com | Common oligo | [P] AAGGTCATCCCTGAGCTGAACG[Biotin] | 23 |
| | G3-Que | Query oligo | [F-9] GGAAGCTCACTGGCATGGCCTTCCGT | 24 |

### 4. Cycling Ligation Reaction

### 4-1. Preparation of Reaction Liquid

The composition in the ligation reaction was as shown in Table 27. As an enzyme for the ligation, Taq DNA Ligase from New England Biolabs Japan Inc. was used.

**[Table 27]**

| Reagent name | Amount per reaction |
|---|---|
| dH₂O | 8.0 µL |
| 10 × Taq DNA ligase buffer | 1.5 µL |
| Common oligo mixtures (1 µM each) | 1.0 µL |
| Query oligo mixtures (5 µM each) | 1.0 µL |
| Taq DNA ligase | 0.5 µL |
| Amplified sample obtained in section 2. | 3.0 µL |
| Total | 15 µL |

### 4-2. Ligation Reaction

A ligation solution was transferred to a PCR tube and set in a thermal cycler. The temperature condition was at 95°C for 1 minute, followed by 58°C for 5 minutes, then 30 cycles at 95°C for 15 seconds and 58°C for 30 seconds, then at 95°C for 1 minute, and the temperature was lowered to 37°C to finish the reaction.

### 5. Detection Using Chromatography Main Body

### 5-1. Hybridization Reaction

A hybridization reaction by nucleic acid chromatography with use of the single-stranded DNA obtained in section 4. and its detection were performed.

### 5-2. Preparation of Reaction Liquid

The hybridization reaction and the detection were performed by using a reaction solution prepared by mixing a developing solution (manufactured by TBA), a latex solution (manufactured by TBA), a TE buffer and a ligation product. The composition of the reaction solution is shown in Table 28.

**[Table 28]**

| Reaction solution composition | |
|---|---|
| Reagent name | Amount per reaction |
| Developing solution (manufactured by TBA) | 10.0 µL |
| Latex solution (manufactured by TBA) | 2.0 µL |
| TE buffer | 3.0 µL |
| Ligation product | 5.0 µL |
| Total | 20.0 µL |

### 5-3. Hybridization Step

To a 1.5 mL tube, 20 µL of each of the above-mentioned reaction solutions was added, and each lower end part of the chromatography main body was inserted to perform a hybridization reaction by chromatography. The hybridization reaction was performed at 37°C. The reaction solution was completely absorbed in about 20 minutes, and the hybridization reaction by chromatography was completed at the stage when all was absorbed. After completion of the reaction, the chromatography main body was air-dried, then the reaction site was visually confirmed, and the image was taken.

### 5-4. Detection Step

The presence or absence of coloring after drying the chromatography main body was visually confirmed. As shown in Fig. 16, known genetic polymorphisms were all matched with the analysis results of genetic polymorphisms by the chromatography main body, and the results in a case where Spacer C3 was present were matched with the results in a case where Spacer C3 was absent. Accordingly, it was shown that, by using the method of the present invention, a sample to be analyzed may be prepared without using a spacer sequence.

## Claims

1. A genetic analysis method comprising hybridizing the following (i) and (ii) and making the hybridized product visually detectable:
(i) an oligonucleotide probe immobilized on a strip;
(ii) a single-stranded ligation product amplified by subjecting the following (a) and (b) to ligation treatment through one or a plurality of thermal cycling treatments using a nucleic acid sample as a template:
(a) a query oligo comprising a first single-stranded oligonucleotide complementary to said nucleic acid sample and a tag sequence capable of hybridizing with said probe or a labeling moiety;
(b) a common oligo comprising a second single-stranded oligonucleotide complementary to said nucleic acid sample and adjacent to said first single-stranded oligonucleotide and a tag sequence or a labeling moiety.

2. The method according to claim 1, further comprising performing a cycling ligation reaction.

3. The method according to claim 1 or 2, wherein the nucleic acid sample and the oligonucleotide are DNA.

4. The method according to any one of claims 1 to 3, wherein the strip is a chromatography main body having a probe region in which a plurality of probes is immobilized at different positions and a position marker region at a position different from the probe region.

5. The method according to any one of claims 1 to 4, for detecting a gene difference.

6. The method according to any one of claims 1 to 5, for detecting a gene polymorphism.

7. A kit for a genetic analysis method according to any one of claims 1 to 6, comprising
a strip on which oligonucleotide probe is immobilized,
a single-stranded oligonucleotide comprising a sequence complementary to a nucleic acid sample and a tag sequence capable of hybridizing with said probe, and
a single-stranded oligonucleotide comprising a sequence complementary to said nucleic acid sample and adjacent to said first single-stranded oligonucleotide and a labeling moiety or a tag sequence.


SEQUENCE LISTING

<110>  KURASHIKI BOSEKI KABUSHIKI KAISHA

<120>  VISUALLY-DETERMINABLE GENE TESTING

<130>  672638

<150>  JP 2015-17396
<151>  2015-01-30

<160>  44

<170>  PatentIn version 3.5

<210>  1
<211>  35
<212>  DNA
<213>  Artificial Sequence

<220>
<223>  forward primer

<400>  1
gacctgactg aggccttcct ggcagagatg gagaa                                35


<210>  2
<211>  38
<212>  DNA
<213>  Artificial Sequence

<220>
<223>  reverse primer

<400>  2
tatgttggag gaggtcaggc ttacaggatc ctggtcaa                             38


<210>  3
<211>  35
<212>  DNA
<213>  Artificial Sequence

<220>
<223>  forward primer

<400>  3
ttggcctttg gaaaatccag tccttcatgc catgt                                35


<210>  4
<211>  41
<212>  DNA
<213>  Artificial Sequence

<220>
<223>  reverse primer

<400>  4
cactgaaacc ctggttatcc cagaaggctt tgcaggcttc a                         41

<210>  5
<211>  30
<212>  DNA
<213>  Artificial Sequence

<220>
<223>  common oligoprove

<400>  5
caggttctca tcattgaagc tgctctcagg                                        30


<210>  6
<211>  15
<212>  DNA
<213>  Artificial Sequence

<220>
<223>  query oligoprobe

<400>  6
agccaccact aagcg                                                        15


<210>  7
<211>  16
<212>  DNA
<213>  Artificial Sequence

<220>
<223>  query oligoprobe


<400>  7
cagccaccac taagca                                                       16


<210>  8
<211>  20
<212>  DNA
<213>  Artificial Sequence

<220>
<223>  common oligoprove

<400>  8
caccaggccc cctgccactg                                                   20


<210>  9
<211>  14
<212>  DNA
<213>  Artificial Sequence

<220>
<223>  query oligoprobe

<400>  9
ggctgcacgc aacc                                                         14


<210>  10
<211>  14

<212>  DNA
<213>  Artificial Sequence

<220>
<223>  query oligoprobe

<400>  10
ggctgcacgc aact                                                          14


<210>  11
<211>  31
<212>  DNA
<213>  Artificial Sequence

<220>
<223>  forward primer

<400>  11
ctgacatctg ctcagccgca acgtacccct g                                       31


<210>  12
<211>  35
<212>  DNA
<213>  Artificial Sequence

<220>
<223>  reverse primer

<400>  12
acctgctgca gcacttcagc ttctcggtgc ccact                                   35


<210>  13
<211>  36
<212>  DNA
<213>  Artificial Sequence

<220>
<223>  forward primer

<400>  13
aagaaaccac ctgcactagg gaggtgtgag catggg                                  36


<210>  14
<211>  35
<212>  DNA
<213>  Artificial Sequence

<220>
<223>  reverse primer

<400>  14
aaacccatct atgcaaatcc tgctcttccg aggcc                                   35


<210>  15
<211>  31
<212>  DNA
<213>  Artificial Sequence

<220>
<223>  forward primer

<400>  15
ggaatgggac tgaggctccc acctttctca t                                    31


<210>  16
<211>  27
<212>  DNA
<213>  Artificial Sequence

<220>
<223>  reverse primer

<400>  16
gcgtcaaagg tggaggagtg ggtgtcg                                         27


<210>  17
<211>  49
<212>  DNA
<213>  Artificial Sequence

<220>
<223>  common oligoprove

<400>  17
tcaccaggaa agcaaagaca ccatgggcat cttgtatgaa gtacccccc                 49


<210>  18
<211>  15
<212>  DNA
<213>  Artificial Sequence

<220>
<223>  query oligoprobe

<400>  18
   cataggggga tgggc                                                       15


<210>  19
<211>  16
<212>  DNA
<213>  Artificial Sequence

<220>
<223>  query oligoprobe

<400>  19
tcataggggg atgggg                                                     16


<210>  20
<211>  44
<212>  DNA
<213>  Artificial Sequence

<220>
<223>  common oligoprove

<400>  20
gtgggtgatg ggcagaaggg ctctagggta tttgcctgct actc                        44


<210>  21
<211>  14
<212>  DNA
<213>  Artificial Sequence

<220>
<223>  query oligoprobe

<400>  21
cgccaaccac accg                                                         14


<210>  22
<211>  14
<212>  DNA
<213>  Artificial Sequence

<220>
<223>  query oligoprobe

<400>  22
cgccaaccac acca                                                         14


<210>  23
<211>  22
<212>  DNA
<213>  Artificial Sequence

<220>
<223>  common oligoprobe

<400>  23
aaggtcatcc ctgagctgaa cg                                                22


<210>  24
<211>  26
<212>  DNA
<213>  Artificial Sequence

<220>
<223>  query oligoprobe

<400>  24
ggaagctcac tggcatggcc ttccgt                                            26


<210>  25
<211>  22
<212>  DNA
<213>  Artificial Sequence

<220>
<223>  forward primer

<400>  25
aataaggact tgtatgaatg gc                                                22

<210>  26
<211>  21
<212>  DNA
<213>  Artificial Sequence

<220>
<223>  reverse primer

<400>  26
ccaacatcga ggtcgtaaac c                                                21


<210>  27
<211>  27
<212>  DNA
<213>  Artificial Sequence

<220>
<223>  common oligoprobe

<400>  27
ccacaggtcc taaactacca aacctgc                                          27


<210>  28
<211>  23
<212>  DNA
<213>  Artificial Sequence

<220>
<223>  query oligoprobe

<400>  28
aatgcaaaca gtacctaaca aac                                              23


<210>  29
<211>  31
<212>  DNA
<213>  Artificial Sequence

<220>
<223>  common oligoprobe


<400>  29
atttaaccat taaggaataa caacaatctc c                                     31


<210>  30
<211>  23
<212>  DNA
<213>  Artificial Sequence

<220>
<223>  query oligoprobe

<400>  30
actaaccaac ccaaagagaa tag                                              23


<210>  31

<211>  34
<212>  DNA
<213>  Artificial Sequence

<220>
<223>  common oligoprobe

<400>  31
aactcaacca caaagggata aaacataact taac                                    34


<210>  32
<211>  28
<212>  DNA
<213>  Artificial Sequence

<220>
<223>  query oligoprobe

<400>  32
ctttaattaa ctattccaaa agttaaac                                          28


<210>  33
<211>  29
<212>  DNA
<213>  Artificial Sequence

<220>
<223>  common oligoprobe

<400>  33
aaccttacac agccccactg ggtccaccc                                         29


<210>  34
<211>  23
<212>  DNA
<213>  Artificial Sequence

<220>
<223>  query oligoprobe

<400>  34
ctttaaaatc acgaccacct tac                                               23


<210>  35
<211>  14
<212>  DNA
<213>  Artificial Sequence

<220>
<223>  common oligoprobe

<400>  35
ataacagcgc aatc                                                         14


<210>  36
<211>  10
<212>  DNA
<213>  Artificial Sequence

<220>
<223>  query oligoprobe

<400>  36
tacccyaggg                                                              10


<210>  37
<211>  18
<212>  DNA
<213>  Artificial Sequence

<220>
<223>  forward primer

<400>  37
   cggttgcatg ctttttgg                                                    18


<210>  38
<211>  20
<212>  DNA
<213>  Artificial Sequence

<220>
<223>  reverse primer

<400>  38
cagcggtatg taaggcgttg                                                   20


<210>  39
<211>  20
<212>  DNA
<213>  Artificial Sequence

<220>
<223>  forward primer

<400>  39
ctgcaccggc tgaaaataag                                                   20


<210>  40
<211>  20
<212>  DNA
<213>  Artificial Sequence

<220>
<223>  reverse primer

<400>  40
atagcccaac aagcaacacc                                                   20


<210>  41
<211>  24
<212>  DNA
<213>  Artificial Sequence

<220>
<223>  common oligoprobe

<400>  41
tgccaaatcc ctgttttcct gacc                                              24


<210>  42
<211>  20
<212>  DNA
<213>  Artificial Sequence

<220>
<223>  query oligoprobe

<400>  42
cgtttcctgc ttgccatgcg                                                   20


<210>  43
<211>  23
<212>  DNA
<213>  Artificial Sequence

<220>
<223>  common oligoprobe

<400>  43
taggccctcg caaacgttct gct                                               23


<210>  44
<211>  20
<212>  DNA
<213>  Artificial Sequence

<220>
<223>  query oligoprobe

<400>  44
attgcgtcgc aagcccacca                                                   20